(19) Europäisches Patentamt
European Patent Office
Office européen des brevets

(11) Veröffentlichungsnummer : **0 404 039 B1**

# (12) EUROPÄISCHE PATENTSCHRIFT

(45) Veröffentlichungstag der Patentschrift : **13.01.93 Patentblatt 93/02**

(21) Anmeldenummer : **90111501.4**

(22) Anmeldetag : **19.06.90**

(51) Int. Cl.$^5$ : **A61K 31/165,** A61K 31/17, A61K 31/495, C07C 275/24, C07C 233/18, C07C 235/34, C07C 235/48, C07D 295/182

## (54) Arzneimittel, enthaltend Di-tert.-Butylhydroxyphenyl-Derivate sowie neue Derivate.

Die Akte enthält technische Angaben, die nach dem Eingang der Anmeldung eingereicht wurden und die nicht in dieser Patentschrift enthalten sind.

(30) Priorität : **23.06.89 DE 3920616**

(43) Veröffentlichungstag der Anmeldung : **27.12.90 Patentblatt 90/52**

(45) Bekanntmachung des Hinweises auf die Patenterteilung : **13.01.93 Patentblatt 93/02**

(84) Benannte Vertragsstaaten : **AT BE CH DE DK ES FR GB GR IT LI LU NL SE**

(56) Entgegenhaltungen :
**DE-A- 1 817 212**
**DE-A- 2 721 398**
**GB-A- 1 405 767**
**GB-A- 1 446 781**
**NL-A- 7 905 000**
**US-A- 3 763 166**
**US-A- 4 066 610**
**US-A- 4 246 198**

(56) Entgegenhaltungen :
**US-A- 4 397 868**
**US-A- 4 473 579**
**US-A- 4 835 189**
**CHEMICAL ABSTRACTS, Band 101, Nr. 6, 6. August 1984, Columbus, Ohio, USA L.P.GUTNIKOVA et al. "Stabilization of T-6 fuelusing urea derivatives of 2,6 -di-tert-butylphenol" Seite 129, rechte Spalte, Zusammenfassung-Nr. 40 727c**

(73) Patentinhaber : **BOEHRINGER MANNHEIM GMBH**
**Sandhofer Strasse 116**
**W-6800 Mannheim 31 (DE)**

(72) Erfinder : **Dreckmann-Behrendt, Bruno, Dr.**
**Dürerstrasse 53**
**W-6800 Mannheim 1 (DE)**
Erfinder : **Witte, Ernst-Christian, Dr.**
**Beethovenstrasse 2**
**W-6800 Mannheim 1 (DE)**
Erfinder : **Wolff, Hans Peter, Dr.**
**Rebenweg 24**
**W-6945 Hirschberg-Grosssachsen (DE)**
Erfinder : **Dresel, Hans Alois, Dr.**
**Mozartstrasse 1**
**W-6905 Schriesheim (DE)**

EP 0 404 039 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Di-tert.-Butylhydroxy-phenyl-Derivate (BHT-Derivate) der allgemeinen Formel I

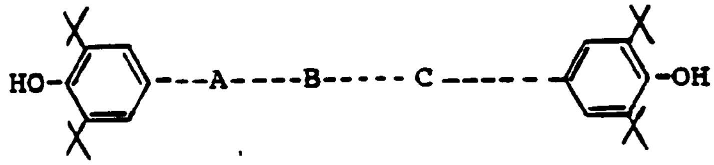

(I)

in der
A und C, die gleich oder verschieden sein können, eine Bindung oder eine gesättigte oder ungesättigte Kohlenwasserstoffkette mit 1-8 C-Atomen, die verzweigt oder unverzweigt sein kann oder die gruppe

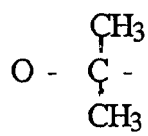 ,

die über das O-Atom an den Phenylring gebunden ist
und B eine der folgenden Gruppen darstellt:

a)

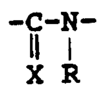

b)

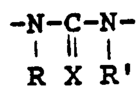

c)

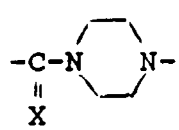

d)

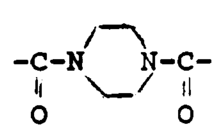

e)

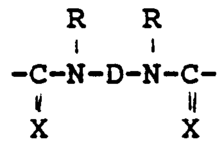

f)

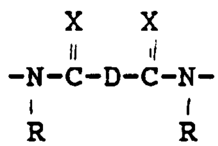

in denen
X ein Sauerstoff- oder Schwefel-atom,
R,R', welche gleich oder verschieden sein können,
ein Wasserstoffatom oder $C_1$-$C_4$ Alkyl, bevorzugt Methyl oder Ethyl, bedeuten,
und
D für eine ungesättigte oder gesättigte, verzweigte oder unverzweigte Kette mit ein bis 10 C-Atomen, oder die Gruppen $-CH_2-CH_2-O-CH_2-CH_2$ oder $-CH_2CH_2-S-CH_2-CH_2-$ steht.

Bevorzugte Bedeutungen von A bzw. C sind Valenz, $-CH_2-CH_2-$,

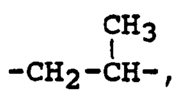,
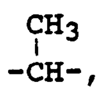,
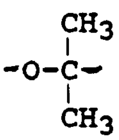

oder -CH=CH-.

D stellt bevorzugt eine $-CH_2-$, $-CH_2-CH_2-$, $-(CH_2)_4-$, $-(CH_2)_6-$, $-CH_2-CH_2-O-CH_2-CH_2-$ oder $-CH_2-CH_2-S-CH_2-CH_2-$ Gruppe dar.

Die Verbindungen der allgemeinen Formel I finden aufgrund ihrer antioxidierenden Eigenschaften, stabilisierenden Wirkung auf Lipoproteine und Hemmwirkung auf die Reacylierung des LDL-Cholesterins in Makrophagen Verwendung als Arzneimittel, insbesondere als Antiarteriosklerotica.

Desweiteren wirken sie antiinflammatorisch, cytoprotektiv sowie antiasthmatisch. Sie können auch als Inhibitoren der Reperfusionsabhängigen Lipidperoxidation und als Stabilisatoren des "lung surfactant factor" eingesetzt werden.

Di-butylierte Hydroxyphenyl-Derivate, welche zwischen zwei Phenylkernen im Spacer Amid-, Ester-, Amin- und Harnstoffstrukturen haben, sind seit längerer Zeit bereits zum Stabilisieren von organischem Material bekannt.

So beschreibt beispielsweise die US-B 3.584.047 Benzamide und Phenylalkancarbonsäureamide sowie die DE-A-27 21 398 (Ciba-Geigy) Phenol-Derivate mit Amidstrukturen in der Seitenkette.

Auch Harnstoffe sowie Thioharnstoffe wurden bereits in der Literatur z.B. als Antioxidantien von T-6 Ölen beschrieben (Oxid. Commun. 5 (1-2), 115 - 28 [CA 102: 9267z] und Neftekhimiya 24 (2), 246 [CA 101: 40727c] sowie Neftekhimiya 27 (5), 703-9) [CA 108: 97496a].

Als Antioxidantien bezeichnet man Substanzen, welche - in geringen Mengen zugegeben - einen zeitweiligen Stillstand oder zumindest eine erhebliche Verzögerung der oxidativen Vorgänge bei dem zu schützenden Produkt bewirken.

Die bisher aus der Literatur bekannten 2,6-Di-tert.-Butylphenyl-Derivate eignen sich als Stabilisator gegen thermooxidativen oder lichtinduzierten Abbau für Polyolefin-Copolymere, Polystyrol, Acryl-Butadien-Styrol,

Polyvinylchlorid, Polyester, Cellulose-Derivate, Elastomere, Klebstoffe, techn. Fette, Mineralöle, Lacke und Farben. Einige Verbindungen wie z.B. Irganox 1076 [3-(3.5-Di-tert.-butyl-4-hydroxyphenyl)-propionsäureoctadecylester] erhielten die FDA-Zulassung für Lebensmittelverpackungen.

Als Pharmakon findet bereits eine Substanz mit einem sterisch gehinderten Alkylphenol Anwendung. Probucol (Lurcelle ®),

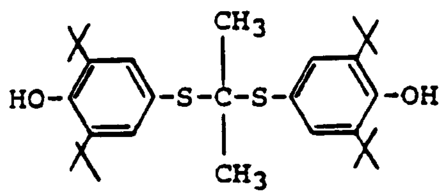

(Probucol)

ursprünglich in der Kunststoff- und Kautschuk- Industrie als Weichmacher bzw. Antioxidans verwendet, zeigt eine hypocholesterinämische Wirkung bei Mäusen, Ratten, Hunden, Affen und am Menschen.

Mit der Reduktion des Serumcholesterins ist eine verminderte Ausscheidung neutraler Sterole, Totalsterole und Gallensäuren in die Faeces verbunden, wobei gleichzeitig die Cholesterin-7-alpha-hydroxylaseaktivität vermindert ist.

Der genaue Wirkmechanismus ist nicht bekannt.

Die im Tierversuch nachweisbare antiatherosklerotische Wirkung von Probucol beruht auf seiner Eigenschaft als Antioxidans: D. Steinberg et al. konnten in in vivo-Experimenten an WHHL-Kaninchen zeigen, daß Probucol die oxidative Modifizierung der LDL-Partikel und die Bildung von cholesterinesterreichen makrophagozytären Schaumzellen in Frühläsionen der Arterienwand verhindert [D. Steinberg et al., N Engl J Med 320, 915 - 924 (1989)].

Die Resorption der stark lipophilen Verbindung ist gering; die Eliminierung aus den Geweben ist äußert langsam, die Ausscheidung erfolgt hauptsächlich über die Faeces [M.N.Cayen, Pharmacol. Ther. 29, 157 (1985)].

Die erfindungsgemäßen Verbindungen sind Stabilisatoren von Lipoprotein-Partikeln und blockieren die Reacylierung von LDL-Cholesterin in Makrophagen. Sie eignen sich daher alleine oder in Kombination mit Antihyperlipidämika zur Prophylaxe und Therapie von Erkrankungen, die durch erhöhtes bzw. durch Oxidation verändertes Serumcholesterin verursacht werden, wie koronare Herzkrankheiten, Atherosclerose, Hyperlipoproteinämie und ähnliche Erkrankungen.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 10 mg/kg Körpergewicht.

Di-tert.-butylierte Hydroxyphenyl-Derivate der allgemeinen Formel I mit einer Amid- oder Harnstoff-gruppe und deren Derivate können nach bekannten Syntheseverfahren erhalten werden, beispielsweise durch die Umsetzung einer Carbonsäure oder eines Derivats (allgemeine Formel II) mit einem Amin der allgemeinen Formel III

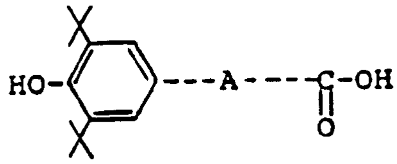

(II),

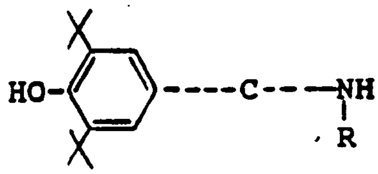

(III),

in denen
A und C sowie der Substituent R die in des allgemeinen Formel I angegebene Bedeutung haben.

Die Umsetzung erfolgt in der Regel in einem inerten Lösungsmittel, wie einem Kohlenwasserstoff, Dimethylformamid oder in Ether.

Vorzugsweise verwendet man äquimolare Mengen an Amin und Carbonsäure in Gegenwart äquimolarer Mengen einer Base, wie einem tert. organ. Amin, bevorzugt Triethylamin, Alkalibicarbonat, Hydroxyd oder -Carbonat.

Als Derivate der Carbonsäure (allgemeine Formel II) können das Säurechlorid oder ein Ester dienen.

Für die Umsetzung der freien Carbonsäure ist in der Regel die Anwesenheit von $POCl_3$, $SOCl_2$ oder $PCl_3$ erforderlich; für die Umsetzung eines Carbonsäure-Esters mit dem Amin ein basischer Katalysator, bevorzugt ein Alkalialkoholat. In vielen Fällen gelingt auch die Reaktion der Carbonsäure mit einem Amin in einem Lösungsmittel wie z.B. Xylol durch Erwärmen unter Rückfluß am Wasserabscheider.

Bei der Darstellung der Diamide werden zwei Äquivalente Carbonsäure (allgemeine Formel II) pro Di-Amin-Komponente (IV) beziehungsweise zwei Äquivalente Amin (III) pro Di-Carbonsäure-Komponente (V) eingesetzt, je nachdem, ob es sich um die Strukturen e) oder f) handelt.

Die Versuchsbedingungen bleiben identisch wie bei den oben beschriebenen Monoamiden.

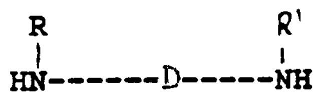

(IV)

$HO_2C$------D-----$CO_2H$ (V)

R, R′ entsprechen der in der allegemeinen Formel I angegebenen Bedeutung.

Das Teilstück Ⓓ entspricht der in der allgemeinen Formel I angegebenen Bedeutung.

Die Harnstoff-Derivate können durch Umsetzung des Amins der allgemeinen Formel III

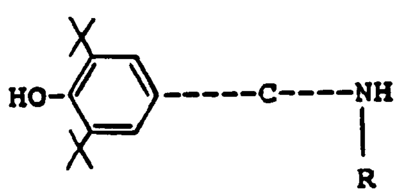

(III)

mit Phosgen dargestellt werden. Dabei wird in einem Überschuß an Amin gearbeitet.

Die entsprechende Umsetzung mit Thiocarbonylchlorid (Thiophosgen) und 4 Äquivalenten des Amins der allgmeinen Formel III führt zum Thioharnstoff. Die Thiocarbamidsäurechloride werden als Zwischenstufe nicht isoliert (vgl. T. J. Pullukat et al., Tetrahedron Lett. 1967, 1953).

Symmetrische Harnstoff-Derivate lassen sich auch durch Carbonylierung von primären Aminen (Aryl- u. Alkylamine) mit Kohlenmonoxid in Gegenwart eines Katalysators, wie Selen oder Schwefel, darstellen [vgl. Sonoda et al., J.Am. Chem.Soc., 93, 6344 (1971) und Franz et al., J.Org.Chem. 26, 3309 (1961)].

Symmetrische Thioharnstoffe entstehen ebenfalls durch Einwirkung von Schwefelkohlenstoff auf Amine, bevorzugt auf aromatische Amine unter Abspaltung von Schwefelwasserstoff. Als Lösungsmittel bewähren sich Alkohol und Aceton [vgl. Houben-Weyl IX, 885 sowie Deposited. Doc., Viniti 6343-82, 93-8, C.A. 100 (19): 156 325 d].

Die für die nachstehenden Beispiele am häufigsten angewandte Methode zur Darstellung von symmetrisch wie auch unsysmmetrisch substituierten Harnstoffen stellt die Addition eines Amins (III) an ein Isocyanat (VI) dar (X = O).

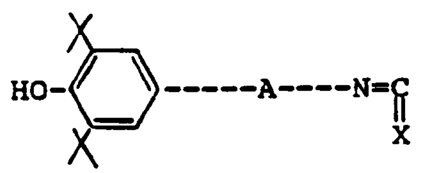

(VI)

X = O,S

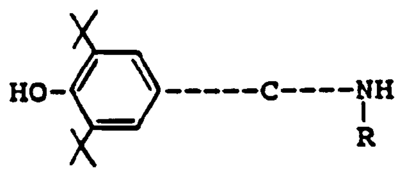

(III)

Die Umsetzungen von Isothiocyanaten (X = S) mit einem primären Amin ergibt das Thioharnstoff-Derivat [analog Satchell et al., Chem. Soc. Rev. 4, 231-50 (1975)].

Diese Methode liefert hervorragende Ausbeuten, wenn in aprotischen Lösungsmitteln, bevorzugt in Kohlenwasserstoffen, oder auch in Ether gearbeitet wird. (Houben-Weyl VIII, 157).

Die in der Erfindung beschriebenen Thioamid- und Thioharnstoffderivate können ebenso aus den Carbonylverbindungen mit Phosphorpentasulfid dargestellt werden.

Man verwendet pro Carbonylfunktion etwa 2 Äquivalente Pentasulfid in wasserfreien aprotischen Lösungsmitteln, bevorzugt Kohlenwasserstoffen wie Xylol.

Als geeigneter erwies sich die Umsetzung mit Lawesson's Reagenz [2,4-Bis-(4-methoxyphenyl)-1,3-dithia-2,4-diphosphetan-2,4-disulfid] in geringem Überschuß (1.1 bis zu 2.0 Äquivalente] in aprotischen Lösungsmitteln, bevorzugt in Toluol oder Xylol bei erhöhten Temperaturen.

Gegenstand der Erfindung sind auch neue Verbindungen der allegemeinen Formel I, insbesondere die folgenden Verbindungen:

Bevorzugte Verbindungen der allgemeinen Formel I:

N-[2-[3,5-Di-tert.-butyl-4-hydroxyphenyl]ethyl]-3,5-di-tert.-butyl-4-hydroxybenzamid
3,5-Di-tert.-butyl-4-Hydroxyphenylessigsäure-(3,5-di-tert.-butyl-4-hydroxyphenyl)methyl-amid
3-(3,5-Di-tert.-butyl-4-hydroxyphenyl)propionsäure-[2-(3,5-di-tert.-butyl-4-hydroxyphenyl)ethyl-amid
3-(3,5-Di-tert.-butyl-4-hydroxyphenyl)zimtsäure-[2-(3,5-di-tert.-butyl)-4-hydroxyphenyl)ethyl]amid
N,N'-Bis-[2-(3,5-di-tert.-butyl-4-hydroxyphenyl)ethyl]harnstoff
N,N'-Bis-[2-(3,5-di-tert.-butyl-4-hydroxyphenyl)ethyl]thioharnstoff
N,N'-Diethyl-N,N'-bis-(3,5-di-tert.-butyl-4-hydroxyphenethyl)hexansäureamid

Die folgenden Beispiele erläutern die Erfindung.

Die Ausgangsstoffe sind bekannt oder können, sofern sie neu sind, in Analogie zu den bekannten Verbindungen in an sich bekannter Weise hergestellt werden.

Beispiel 1

3-(3,5-Di-tert.butyl-4-hydroxyphenyl)-propionitril

Ein Gemisch aus 206.3 g (1 mol) 2,6-Di-tert.-butylphenol, 4.8 g pulv. Natriumborhydrid und 262 ml (4 mol) Acrylnitril wird 16 h im Ölbad auf 90°C erwärmt (leichter Rückfluß). Man kühlt ab, rührt in 1 l Wasser ein, säuert an und gibt 900 ml Methylenchlorid zu. Danach saugt man das gebildete Produkt ab, trennt, wäscht die Wasserphase zweimal mit wenig Methylenchlorid, vereint die Methylenchloridphasen, trocknet sie und engt ein. Das Rohprodukt wird auf der Nutsche zweimal mit je 250 ml Ligroin gewaschen.
Ausbeute: 203.6 g (78 % d. Theorie)
Schmp. 110-113°C
Lit.: Sumitomo, Jap. Kokai 75, 71 638

Beispiel 2

3-(4-Hydroxy-3,5-di-tert.-butyl-phenyl)-propionsäure

Ein Gemisch aus 190 ml Wasser, 148.0 g (3.7 mol) NaOH, 800 ml Ethanol und 95.6 g (0.37 mol) des Propionitrils (s.o.) wird unter Stickstoff 18 h auf Rückflußtemperatur erhitzt. Dann kühlt man ab, säuert mit 6 N Salzsäure auf pH 2 an, saugt ab und wäscht den Filterkuchen mit Wasser. Nach Umkristallisation aus 66proz. Alkohol erhält man 95.0 g (92 % d. Theorie) Carbonsäure.
Schmp.: 169.5-170°C

Beispiel 3

4-Hydroxy-3,5-di-tert.butyl-benzylchlorid

Man löst 52.8 g (0.256 mol) 2,6-Di-tert.-butylphenol in 200 ml n-Heptan, gibt 250 ml 37proz. Formalinlösung und 500 ml konz. Salzsäure zu, spült mit Stickstoff und rührt 18 h bei Rückflußtemperatur. Nach dem Abkühlen trennt man die org. Phase ab, extrahiert die wässrige Phase mit n-Heptan und wäscht die vereinten Heptanphasen mit Wasser. Nach Trocknen mittels $Na_2SO_4$ wird i. Vak. eingedampft und der Rückstand i. Vak. destilliert.
Sdp.: 132-134°C/0.01 Torr
Ausbeute: 46.2 g (71 % d. Theorie)
Lit.: Neureither, J.Org.Chem. 28, 3486 (1963)

Beispiel 4

4-Hydroxy-3,5-di-tert.-butylbenzylcyanid

Zu einer 80-85°C heißen Mischung aus 38.4 g (0.71 mol) Natriumcyanid, 50 ml Wasser und 72 ml Ethanol tropft man innerhalb 1 h eine Lösung aus 104.5 g (0.41 mol) 4-Hydroxy-3,5-di-tert.-butylbenzylchlorid und 155 ml Ethanol. Anschließend hält man weitere 3 h auf Rückflußtemperatur, kühlt ab und saugt anorgan. Material ab. Die flüssige Phase wird eingedampft, der Eindampfrückstand mit Wasser versetzt und mit Ether extrahiert. Man trocknet die Etherphase ($Na_2SO_4$), dampft ein und bringt durch Ligroin-Zugabe zur Kristallisation.
Ausbeute: 61.1 g (61 % d. Theorie)
Schmp.: 109-110°C
analog Fuson und Rabjohn, Org. Synth. Vol. 25, 66.

Beispiel 5

4-Hydroxy-3,5-di-tert.-butylphenethylamin

Zu einer Suspension aus 19.0 g (0.5 mol) $LiAlH_4$ und 600 ml absol. Ether tropft man unter Stickstoff eine Lösung aus 60.0 g (0.245 mol) 4-Hydroxy-3,5-di-tert.-butylbenzylcyanid und 400 ml absol. Ether. Man rührt weitere 4 h bei Rückflußtemperatur, zersetzt dann mit Natronlauge und Wasser und saugt ab. Die Etherphase wird getrocknet mit Natriumsulfat und eingedampft.
Ausbeute: 59 g (97 % d. Theorie)

Schmp.: 106-108°C (Umkrist. aus Ligroin).

Beispiel 6

3-(3,5-Di-tert.-butyl-4-hydroxyphenyl)-propionsäure-[2-(3,5-di-tert.-butyl-4-hydroxyphenyl)-ethyl]-amid

Eine Lösung von 7.48 g (0.03 mol) 3-(4-Hydroxy-3,5-di-tert.-butylphenyl)propionsäure und 8.35 g (0.03 mol) 4-Hydroxy-3,5-di-tert.-butylphenethylamin in 250 ml Xylol wird 56 h am Wasserabscheider unter Rückfluß erhitzt; weder Carbonsäure noch Amin sind nach dieser Zeit dünnschichtchromatographisch nachweisbar. Der Ansatz wird eingeengt, der Rückstand kristallisiert durch Verreiben mit Ligroin.
Ausbeute: 10.9 g (71 % d. Theorie)
Schmp.: 172°C

Beispiel 7

3,5-Di-tert.-butyl-4-hydroxy-zimtsäure

Eine Lösung von 75.5 g (0.3 mol) 3,5-Di-tert.-butyl-4-hydroxybenzaldehyd, 270 ml Pyridin, 10 ml Piperidin und 100 g (0.95 mol) Malonsäure wird 2 h auf 85°C unter Stickstoff unter Rühren erwärmt; anschließend rührt man schnell in 750 g Eis und 150 ml konz. Salzsäure ein. Das entstehende Öl nimmt man in Methylenchlorid auf, extrahiert viermal mit 2 N Salzsäure, wäscht neutral, trocknet über Natriumsulfat und dampft i. Vak. ein. Nach Umkristallisation erhält man das Zimtsäurederivat als Kristallisat mit einem Schmelzpunkt von 198-201°C
Ausbeute: 59 %.

Beispiel 8

3-(3,5-Di-tert.-butyl-4-hydroxyphenyl)-zimtsäure-[2-(3,5-di-tert.-butyl-4-hydroxyphenyl)-ethyl]-amid

Zu einer Lösung aus 4.44 g (0.016 mol) 3,5-Di-tert.-butyl-4-hydroxyzimtsäure und 4.0 g (0.016 mol) 4-Hydroxy-3,5-di-tert.-butylphenethylamin in 100 ml Pyridin gibt man 1.1 g (0.008 mol) Phosphortrichlorid und erwärmt 10 h auf 90°C.

Nach dem Einengen wird der Rückstand in Ether aufgenommen, mehrmals mit 0.5 N Natronlauge, anschließend mit 0.5 N Salzsäure und Wasser gewaschen und eingeengt.
Ausbeute: 71 %
Schmp.: 245°C

Beispiel 9

3,5-Di-tert.-butyl-4-hydroxy-phenethyl-isocyanat

Eine Lösung aus 24.94 g (0.1 mol) 3,5-Di-tert.-butyl-4-hydroxy-phenethylamin und 100 ml absol. Toluol wird zunächst bis zur sauren Reaktion mit Chlorwasserstoff begast. Dann erhitzt man auf 100°C und leitet langsam 49.4 g (0.5 mol) Phosgen ein. Anschließend hält man 45 min auf Rückflußtemperatur. Nach Stehen über Nacht wird i. Vak. das Toluol abdestilliert, wobei man das Isocyanat als schwarzes Öl erhält, das ohne weitere Aufreinigung für die Harnstoff-Synthese verwendet werden kann.

Beispiel 10

N,N'-Bis-(3,5-di-tert.-butyl-4-hydroxy-phenylethyl)-harnstoff

Zu einer Lösung aus 19.9 g (80 mmol) 3,5-Di-tert.-butyl-4-hydroxyphenethylamin in 100 ml absol. Ether tropft man unter Stickstoff eine Lösung aus 22 g (80 mmol) des rohen Isocyanats und 40 ml absol. Ether. Nachdem die schwach exotherme Reaktion abgeklungen ist, fällt ein kristalliner Niederschlag aus, der abgesaugt, getrocknet und zweimal aus Ethanol umkristallisiert wird.
Ausbeute: 27.8 g, (66 % d. Theorie)
Schmp.: 191-192°C

Beispiel 11

2-(3,5-Di-tert.-butyl-4-hydroxy-phenoxy)-2-methyl-propionsäure

Unter Stickstoff mischt man 50.0 g (225 mmol) 2,6-Di-tert.-butyl-hydrochinon mit 500 ml Butanon-2 und 48.4 g (350 mmol) Kaliumcarbonat, rührt 2 h bei 80°C, gibt eine Spatelspitze Kaliumjodid sowie 68.2 g (350 mmol) 2-Brom-2-methylpropionsäureethylester zu und hält 4 h auf Rückflußtemperatur. Man saugt ab, dampft i. Vak. ein und destilliert den Rückstand im Hochvakuum. Zwischen 143°C und 144°C/0.005 Torr erhält man 50.0 g (66 % d. Theorie) farbloses, langsam erstarrendes Produkt, das im Kältebad aus Ligroin umkristallisiert wird.
Schmp.: 50-51°C

17.0 g (50 mmol) des Ethylesters werden in 125 ml Ethanol und 125 ml 2 N Natronlauge (0.25 mol) 30 min auf Siedetemperatur erwärmt, nach dem Abkühlen mit 125 ml 2 N Salzsäure (0.25 mol) versetzt. Der ausgefallene Niederschlag ergibt 10.7 g (69 % d. Theorie) Säure mit dem Schmp. 122-123°C.

Beispiel 12

N,N'-Bis-(3,5-di-tert.-butyl-4-hydroxyphenethyl)-thioharnstoff

Zu einer Lösung von 2.62 g (5 mmol) N,N'-Bis-(3,5-di-tert.-butyl-4-hydroxyphenethyl)-harnstoff in 20 ml absol. Toluol gibt man unter Stickstoff 1.2 g (3 mmol) Lawesson's Reagenz [2,4-Bis-(4-methoxyphenyl)-1,3-dithia-2,4-diphosphetan-2,4-disulfid].

Danach erwärmt man auf 100°C und läßt 1.5 h bei dieser Temperatur rühren. Nach dem Abkühlen wird die Lösung eingeengt und der Rückstand mehrmals aus Ether/Isohexan umkristallisiert.
Ausbeute: 63 %
Schmp.: 135-136°C

Beispiel 13

In analoger Weise wie in den Beispielen 6, 8, 10 bzw. 12 beschrieben erhält man:

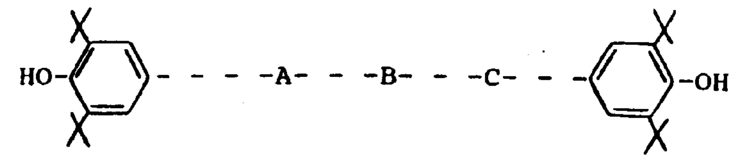

| Nr. | - - -A- - -B- - -C- - - | Schmp. |
|---|---|---|
| 1 | $-CO-NH-CH_2-$ | 233°C |
| 2 | $-CH_2-CO-NH-$ | 225°C |
| 3 | $-CH_2CH_2-CO-NH-CH_2CH_2-$ | 172°C |
| 4 | $-CO-NH-$ | 302°C |
| 5 | $-CH_2CH_2-NH-CO-$ | 230°C |
| 6 | $-CH_2-CO-NH-CH_2-$ | 175-178°C |
| 7 | $-CH=CH-CO-NH-CH_2CH_2-$ | 245°C |

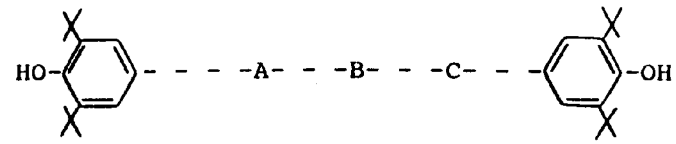

| Nr. | - - -A- - -B- - -C- - - | Schmp. |
|---|---|---|
| 8 | $-CH_2CH_2-NH-CO-NH-CH_2CH_2-$ | 191°C |
| 9 | $-CH_2CH_2-NH-CS-NH-CH_2CH_2-$ | 135-136°C |
| 10 | $CH_3$ $CH_3$<br>$-CH_2-C-NH-CO-CH_2CH_2-S-CH_2CH_2-CO-NH-C-CH_2-$<br>$CH_3$ $CH_3$ | 169°C |
| 11 | $CH_3$<br>$-CH_2-C-NH-CO-CH_2CH_2-$<br>$CH_3$ | 177°C |
| 12 | $CH_3$ $CH_3$<br>$-CH_2-C-NH-CO-(CH_2)_4-CO-NH-C-CH_2-$<br>$CH_3$ $CH_3$ | 235°C |
| 13 | $CH_3$ $CH_3$<br>$-CH_2-C-NH-CO-(CH_2)_2-CO-NH-C-CH_2-$<br>$CH_3$ $CH_3$ | 138°C |
| 14 | | |

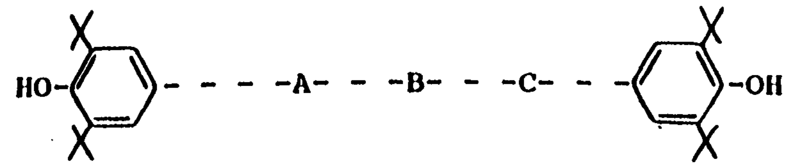

| Nr. | - - -A- - -B- - -C- - - | Schmp. |
|---|---|---|
| 15 | 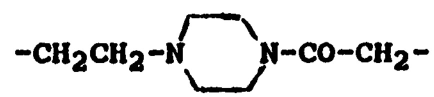 | HCl:274-6 Zers. Ba:207-8°C |
| 16 | 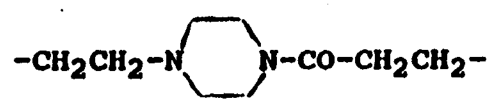 | Ba:205°C Z. |
| 17 | 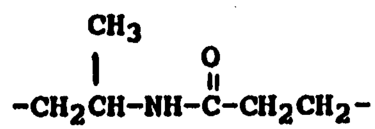 | 154°C |
| 18 | 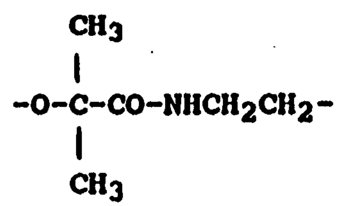 | 153-154°C |

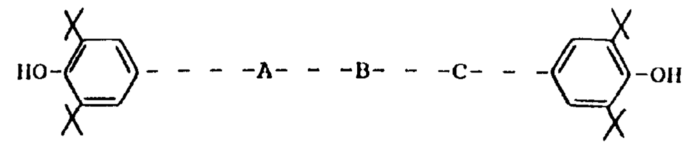

| Nr. | - - -A- - -B- - -C- - - | Schmp. |
|---|---|---|
| 19 | $-CH_2-CO-NH-CH_2CH_2-$ | 163°C |
| 20 | $CH_3$<br>\|<br>$-CH-CO-NH-CH_2CH_2-$ | 202°C |
| 21 | $-CS-NH-$ | 165-166°C |
| 22 | $-CH{=}CH-CS-NH-CH_2CH_2-$ | 244°C |
| 23 | $-CH_2CH_2-NH-CS-$ | 202-203°C |
| 24 | $-CH_2CH_2-NH-CS-CH_2CH_2-$ | 185°C |

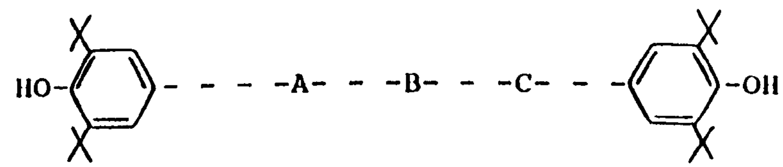

| Nr. | - - -A- - -B- - -C- - - | Schmp. |
|---|---|---|
| 25 | $-CH_2CH_2-N-CO(CH_2)_4CO-N-CH_2CH_2-$<br>\| \|<br>$C_2H_5$ $C_2H_5$ | 175°C |
| 26 | $C_2H_5$<br>\|<br>$-CH_2CH_2-N-COCH_2CH_2-$ | 128-130°C |
| 27 | $C_2H_5$ $C_2H_5$<br>\| \|<br>$-CH_2CH_2CO-N-CH_2CH_2-N-COCH_2CH_2$ | 183°C |

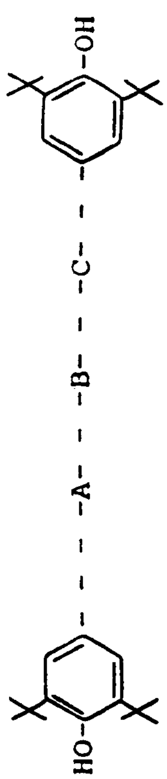

| Nr. | - - -A- - -B- - -C- - - | Schmp. |
|---|---|---|
| 28 | $-CH_2CH_2NHCOCH_2CONHCH_2CH_2-$ | 202°C (Zers.) |
| 29 | $-CH_2CH_2NHCO(CH_2)_4CONHCH_2CH_2-$ | 187°C |
| 30 | $-CH_2CH_2CONH(CH_2)_6NH-CO-CH_2CH_2-$ | 156°C |
| 31 | $-CH_2CH_2CONH(CH_2)_4NHCOCH_2CH_2-$ | 203°C |

## Patentansprüche

1. Arzneimittel, enthaltend eine Verbindung der allgemeinen Formel I

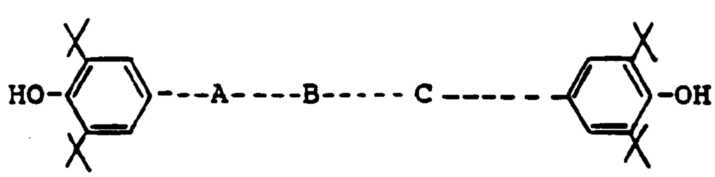

(I)

in der
A und C, die gleich oder verschieden sein können, eine Bindung oder eine gesättigte oder ungesättigte Kohlenwasserstoffkette mit 1-8 C-Atomen, die verzweigt oder unverzweigt sein kann oder die Gruppe

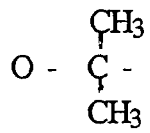 ,

die über das O-Atom an den Phenylring gebunden ist
und B eine der folgenden Gruppen darstellt:

a)

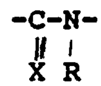

b)

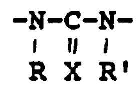

2. Verwendung von Verbindungen gemäß Anspruch 1 als Antiarteriosklerotica,

3. Verbindungen ausgewählt aus der Gruppe:
N-[2-[3,5-Di-tert,-butyl-4-hydroxyphenyl]ethyl]-3,5-di -tert,-butyl-4-hydroxybenzamid
3,5-Di-tert,-butyl-4-Hydroxyphenylessigsäure-(3,5-di-tert.-butyl-4-hydroxyphenyl)methyl-amid
3-(3,5-Di-tert,-butyl-4-hydroxyphenyl)propionsäure-[2-(3,5-di-tert.-butyl-4-hydroxyphenyl)ethyl-amid
3-(3,5-Di-tert,-butyl-4-hydroxyphenyl)zimtsäure-[2-(3,5-di-tert.-butyl)-4-hydroxyphenyl)ethyl]amid
N,N′-Bis-[2-(3,5-di-tert.-butyl-4-hydroxyphenyl)ethyl]-harnstoff
N,N′-Bis-[2-(3,5-di-tert,-butyl-4-hydroxyphenyl)ethyl]-thioharnstoff
N,N′-Diethyl-N,N′-bis-(3,5-di-tert,-butyl-4-hydroxyphenethyl)hexansäureamid.

c)

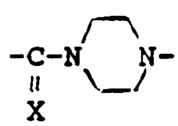

d )

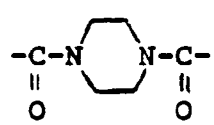

e)

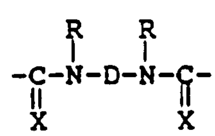

f)

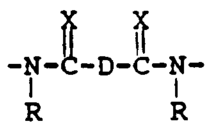

in denen

X ein Sauerstoff- oder Schwefel-atom,
R, R′, die gleich oder verschieden sein können,
ein Wasserstoffatom oder $C_1$-$C_4$ Alkyl bedeuten,
und
D für eine ungesättigte oder gesättigte, verzweigte oder unverzweigte Kette mit ein bis 10 C-Atomen, oder die Gruppen $-CH_2-CH_2-O-CH_2-CH_2-$ oder $CH_2-CH_2-S-CH_2-CH_2-$, steht, neben üblichen Träger- und Hilfsstoffen.

## Claims

1. Medicament containing a compound of the general formula I

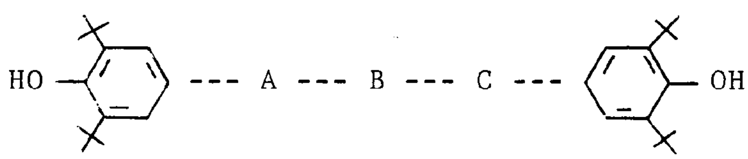 (I)

in which A and C, which can be the same or different, signify a valency bond or saturated or unsaturated hydrocarbon chain with 1 - 8 C-atoms, which can be branched or unbranched or the group

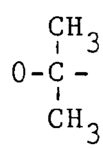

which is connected via the O-atom to the phenyl ring and B represents one of the following groups:

a) 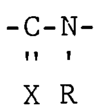 , b) 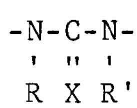 , c) 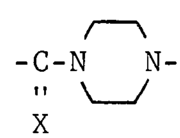 ,

d) 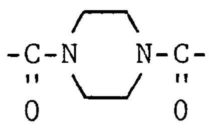 , e) 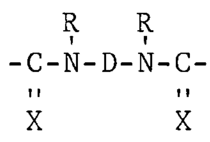 , f) 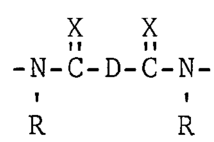

in which X signifies an oxygen or sulphur atom, R, R′, which can be the same or different, signify a hydrogen atom or $C_1$-$C_4$-alkyl and D stands for an unsaturated or saturated, straight-chained or branched chain with one to 10 carbon atoms or the groups $-CH_2-CH_2-O-CH_2-CH_2-$ or $-CH_2-CH_2-S-CH_2-CH_2-$, as well as usual carrier and adjuvant materials.

2. Use of compounds according to claim 1 as antiarteriosclerotics.

3. Compounds selected from the group:
N-[2-[3,5-di-tert.-butyl-4-hydroxyphenyl]-ethyl]-3,5-di-tert.-butyl-4-hydroxybenzamide
3,5-di-tert.-butyl-4-hydroxyphenylacetic acid (3,5-di-tert.-butyl-4-hydroxyphenyl)-methylamide
3-(3,5-di-tert.-butyl-4-hydroxyphenyl)-propionic acid [2-(3,5-di-tert.-butyl-4-hydroxyphenyl)-ethylamide
3-(3,5-di-tert.-butyl-4-hydroxyphenyl)-cinnamic acid [2-(3,5-di-tert.-butyl)-4-hydroxyphenyl]-ethylamide
N,N'-bis-[2-(3,5-di-tert.-butyl-4-hydroxyphenyl)ethyl]-urea
N,N'-bis-[2-(3,5-di-tert.-butyl-4-hydroxyphenyl)ethyl]-thiourea
N,N'-diethyl-N,N'-bis-(3,5-di-tert.-butyl-4-hydroxyphenethyl)-hexanoic acid amide

**Revendications**

1. Médicament, contenant un composé de formule générale I

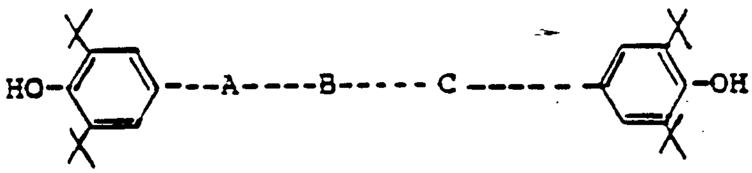

(I)

dans laquelle
A et C qui peuvent être identiques ou différents, représentent une liaison directe ou une chaîne hydrocarbonée saturée ou insaturée, comportant 1 -8 atomes de carbone, qui peut être droite ou ramifiée, ou le groupe

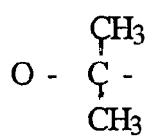,

qui est lié au noyau phényle par l'intermédiaire de l'atome de O,
et
B représente un des groupes suivants :
a)

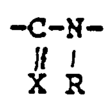

b)

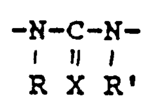

c)

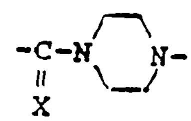

d)

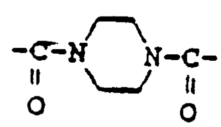

e)

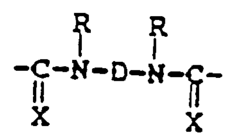

f)

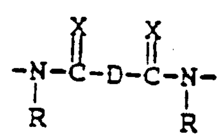

dans lesquels

X représente un atome d'oxygène ou un atome de soufre,
R, R' qui peuvent être identiques ou différents, représentent un atome d'hydrogène ou un groupe alkyle en $C_1$-$C_4$,
et
D représente une chaîne saturée ou insaturée, linéaire ou ramifiée, comportant 1 à 10 atomes de carbone, ou les groupes $-CH_2-CH_2-O-CH_2-CH_2-$ ou $-CH_2-CH_2-S-CH_2-CH_2-$, en plus des supports et adjuvants usuels.

**2.** Utilisation de composés selon la revendication 1, comme agent anti-artériosclérose.

**3.** Composés choisis dans le groupe comprenant les composés suivants :
N-[2-[3,5-di-tert.-butyl-4-hydroxyphényl]éthyl]3,5-di-tert.-butyl-4-hydroxybenzamide
(3,5-di-tert.-butyl-4-hydroxyphényl)méthylamide de l'acide 3,5-di-tert.-butyl-4-hydroxyphénylacétique
[2-(3,5-di-tert.-butyl-4-hydroxyphényl)éthyl]amide de l'acide 3-(3,5-di-tert.-butyl-4-hydroxyphényl)propionique
[2-(3,5-di-tert.-butyl)-4-hydroxyphényl)éthyl]amide de l'acide 3-(3,5-di-tert.-butyl-4-hydroxyphényl)cinnamique
N,N'-bis-[2-(3,5-di-tert.-butyl-4-hydroxyphényl)éthyl]urée
N,N'-bis-[2-(3,5-di-tert.-butyl-4-hydroxyphényl)éthyl]thiourée
amide de l'acide N,N'-diéthyl-N,N'-bis-(3,5-di-tert.-butyl-4-hydroxyphénéthyl)hexanoïque